# EUROPEAN PATENT APPLICATION

(11) **EP 1 905 758 A1**
(43) Date of publication of application: **02.04.2008**
(21) Application number: 07020624.8
(22) Date of filing: 17.10.2002
(51) Int. Cl.: C07C 303/44, C07C 217/54, C07C 311/37

(54) **Tamsulosin free base and recovery process thereof**

(30) Priority: 31.10.2001 US 330817 P; 31.07.2002 US 208009
(62) Divisional of application: 02768175.8
(71) Applicant: Synthon B.V., 6545 CM Nijmegen (NL)
(72) Inventor: Hoorn, Hans Jan, 6537 TG Nijmegen (NL); Peters, Theodorus Hendricus Antonius, 6814 JB Arnhem (NL); Pis, Jaroslav, 100 00 Praha 10 (CZ)
(74) Representative: Duxbury, Stephen

(57) **Abstract**

Racemic tamsulosin free base is obtained in solid form. The solid form can be formed by precipitating racemic tamsulosin free base from a solvent that comprises at least one of water or a lower alcohol. The crystalline free base exhibits polymorphism and two unique forms are identified. Novel intermediates are disclosed useful in the production of tamsulosin free base: a sulfonic acid salt of 2-(o-ethoxyphenoxy)ethylamine and (3-aminosulfonyl-4-methoxy)phenylacetone.

## Description

### BACKGROUND OF THE INVENTION

The present invention relates to (R,S) -5-[2-[[2-(2-ethoxyphenoxy)ethyl]amino]propyl]-2-methoxybenzene-sulfonamide, also known as tamsulosin, in solid state and methods of making the same.

The compound 5-[2-[[2-(2-ethoxyphenoxy)ethyl]amino]propyl]-2-methoxy-benzenesulfonamide of the formula (1) 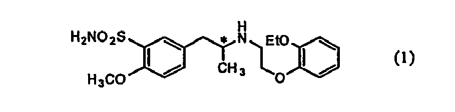 is a commercially marketed pharmaceutically active substance useful for treatment of cardiac insufficiencies and benign prostatic hyperplasia. It is disclosed in EP34432 and US 4731478. The molecule, which will be further denoted as "tamsulosin," has one asymmetric carbon (indicated by an asterisk in the above formula (1)), thus allowing for the existence of two enantiomers, conventionally denoted as (R)- or (S)-enantiomers. Both the free base and its acid addition salts thereof may comprise either one or both of the two enantiomers. The single enantiomers have distinctive optical activity in polarized light and they also differ in their pharmacologic activities. The commercially marketed product is the hydrochloride salt of the (R)-enantiomer of tamsulosin which is levorotary or (R)(-) tamsulosin hydrochloride.

EP34432/ US 4731478 discloses two general processes that may provide tamsulosin. One general process (hereinafter "process A") comprises a reductive amination of a benzylmethylketone compound with a substituted phenoxyethylamine. To make tamsulosin, the corresponding species would be represented by formulae (4) and (5), respectively.

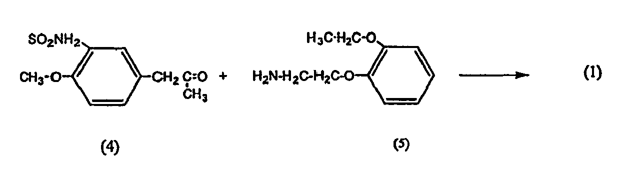

However, neither these compounds nor the details of an actual production process leading to tamsulosin using this process is set forth. Instead, process A was exemplified only for alkylsulfonamide derivatives. Accordingly, the compounds (4) and (5) were also not prepared as chemical entities. Further, the exemplified products of process A, see examples 4 and 5, were crystallized as the hydrochloride salt and not as the free base. If process A is applied for the synthesis of tamsulosin, the result would allow for producing a racemic tamsulosin, however isolated in the form of a hydrochloride salt and not as the free base.

The second process (hereinafter "process B") generically teaches conversion of a hydroxylated analogue into the desired sulfonamide via a chloro-analogue. For making tamsulosin, the hydroxy analogue would be a compound of formula (8). 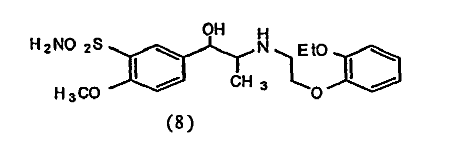 It was disclosed therein that the starting hydroxy-analogues may be prepared according to the methods of GB 2006772, which correspond to DE 2843016 and US 4217305. However, none of these documents specifically show the formation of this hydroxy-tamsulosin intermediate compound. Further, it appears in both US 4731478 and GB 2006772 that only racemic tamsulosin or racemic hydroxy-tamsulosin, respectively, would be prepared by the disclosed methods. None of the examples in US 4731478 show the details of formation of tamsulosin via this process. Moreover, the related compounds made by process B in Examples 1-3 of US 4731478 are isolated as HCl salts via crystallization and not as the free base.

While US 4731478 does disclose racemic tamsulosin HCl in Example 20, it does not show how the compound was made. Apparently the compound was isolated as the HCl salt and not as the free base.

A third process (hereinafter "process C") was disclosed only in the examples of US 4731478, and not in the text, that comprises reacting optically pure 5-((2-amino-2-methyl)ethyl)-2-methoxybenzenesulfonamide with 2-(o-ethoxyphenoxy)ethyl bromide to form the corresponding (R)- or (S)-tamsulosin. See Examples 33(a) and 33(b). In this process the single enantiomeric tamsulosin free base was isolated from CHCl₃-methanol (9:5) as crude crystals and then converted to the HCl salt. Known processes for providing the optically pure amine, e.g. processes disclosed in JP 58-18353, EP257787, JP 02-679248, are lengthy and complicated. Further, if the optical purity is inadvertently insufficient in the amine, then the resulting tamsulosin will likewise be optically impure. There is no known method for purifying an optically impure tamsulosin.

Accordingly, the above described prior art does not teach forming racemic tamsulosin free base in solid state, nor provide any impetus to form such a material.

### SUMMARY OF THE INVENTION

The present invention relates to tamsulosin in solid state. One aspect of the present invention relates to racemic tamsulosin free base in solid state. Typically the free base is a precipitate and is preferably in crystalline form. Crystalline tamsulsoin free base of the present invention exhibits polymorphism and two particular polymorphic forms, hereinafter identified as Form 1 and Form 2, represent preferred aspects of the present invention.

Another aspect of the present invention relates to a process for producing racemic tamsulosin free base in solid state, which comprises precipitating racemic tamsulosin free base from a solution containing racemic tamsulosin free base in a solvent, wherein the solvent comprises at least one of water or a lower alcohol. Preferred solvents include water, methanol, water/methanol mixtures and ethyl acetate/methanol mixtures. The tamsulosin solution can be formed by dissolving a tamsulosin residue into the solvent or by treating a tamsulosin acid addition salt with a base.

Isolation of tamsulosin free base in solid state is not limited to the racemate, however, and another aspect of the present invention relates to a process for isolation of tamsulosin free base, which comprises treating an acid addition salt of tamsulosin in a solvent with a base and precipitating tamsulosin free base from the solvent, wherein the solvent comprises water, a lower alcohol or both. The tamsulosin free base can be racemic or not. The acid addition salt is normally the hydrochloride salt but is not limited thereto and includes, for example, the camphor-10-sulfonic acid salt thereof.

An additional aspect of the present invention relates to a sulfonic acid salt of 2-(o-ethoxyphenoxy)ethylamine, especially the methane sulfonate or tosylate salts thereof, as well as to (3-aminosulfonyl-4-methoxy)phenylacetone. These compounds are novel intermediates that are useful in the production of tamsulosin free base.

### BRIEF DESCRIPITION OF THE DRAWINGS

Fig. 1 is an X-ray diffraction pattern of racemic tamsulosin free base Form 1.
Fig. 2 is an X-ray diffraction pattern of racemic tamsulosin free base Form 2.
Fig. 3 is an IR spectrum of racemic tamsulosin free base Form 1 in KBr.
Fig. 4 is an IR spectrum of racemic tamsulosin free base Form 2 in KBr.
Fig. 5 is a DSC scan of racemic tamsulosin free base Form 1.
Fig. 6 is a DSC scan of racemic tamsulosin free base Form 2.

### DETAILED DESCRIPTION OF THE INVENTION

Racemic tamsulosin free base in solid state was not prepared in any of the above described prior art. The present invention is based in part on the discovery that the racemic free base can be formed and isolated in the solid state. Preferably the solid state form is a precipitate from a solution. More preferably, the solid state form is a crystalline form. The crystalline form includes all polymorphic modifications, unless otherwise noted and includes hydrates and solvates thereof. In particular, two polymorphic forms of solid state racemic tamsulosin free base, designated as Form 1 and Form 2, have now been discovered as part of the present invention and are described hereinafter. The solid state racemic free base can be isolated in high purity, including at least 80% purity, more preferably at least 90% purity, still more preferably at least 95% purity. Relatively pure precipitates are white or almost white microcrystalline substances, are sparingly soluble in water, are soluble in alcohols such as methanol and ethanol, and are soluble in chlorinated hydrocarbons.

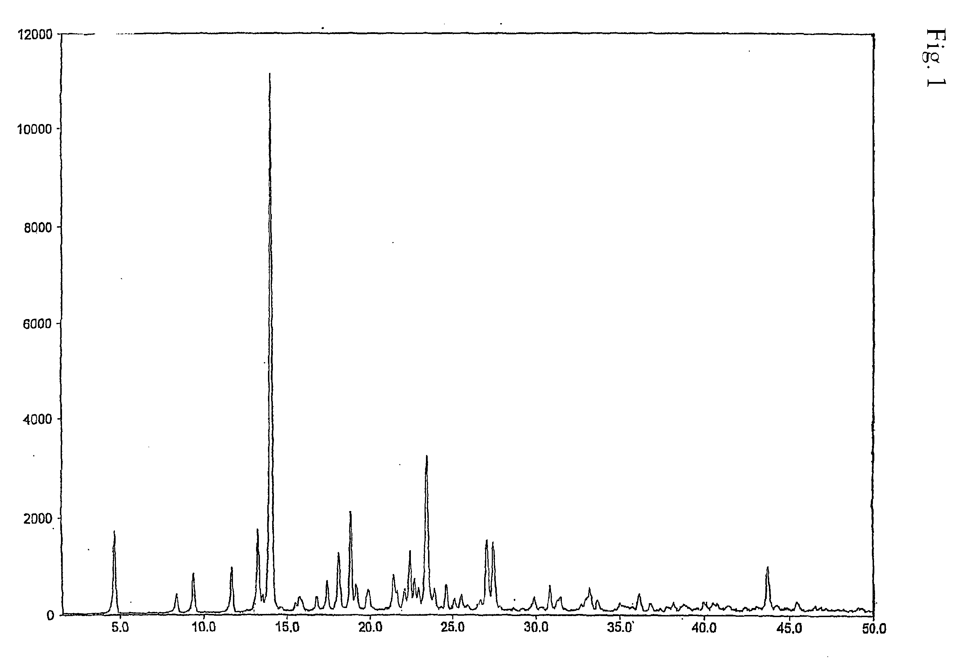

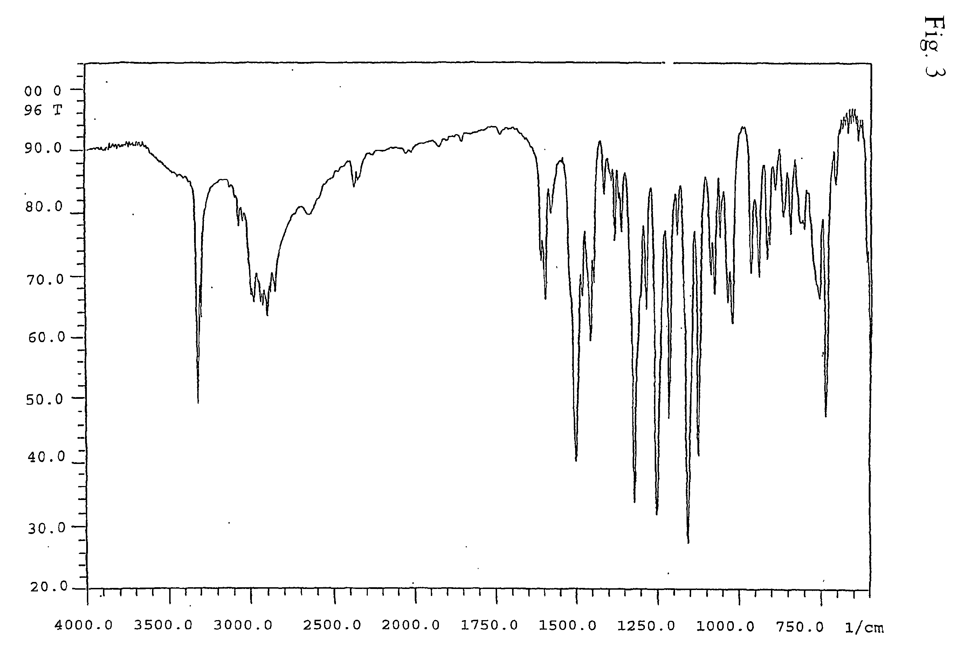

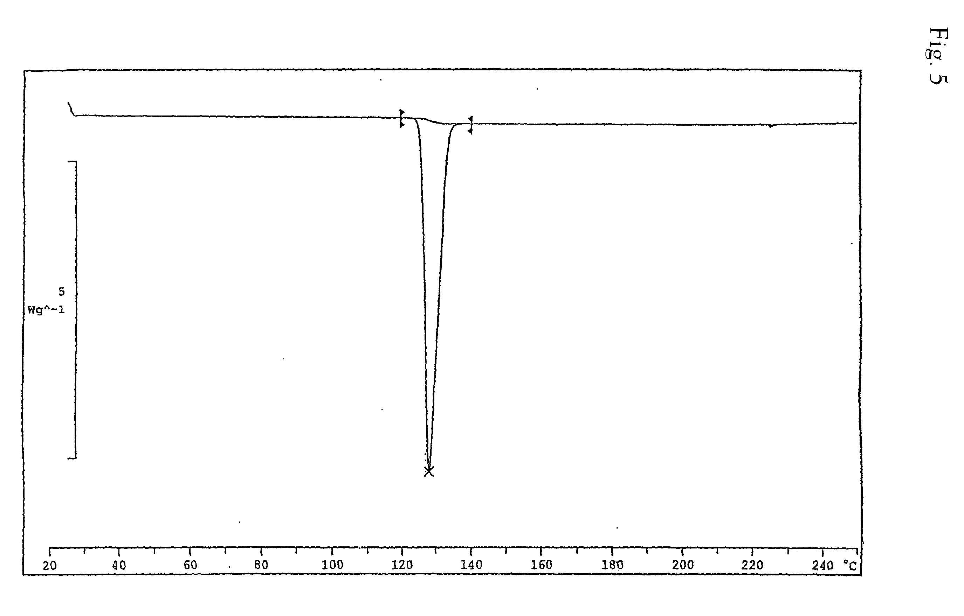

Racemic tamsulosin free base may exist in various polymorphic modifications. One such modification is designated herein as Form 1. This Form of the free base has an X-ray diffraction pattern that substantially corresponds to Fig 1, an IR absorbance spectrum that substantially corresponds to Fig 3, and a DSC curve that substantially corresponds to Fig 5. Form 1 generally has a melting point of about 127-129°C. The Form 1 polymorph is obtainable, *inter alia*, by crystallization of tamsulosin free base from methanol/ethyl acetate mixture, but is not limited thereto.

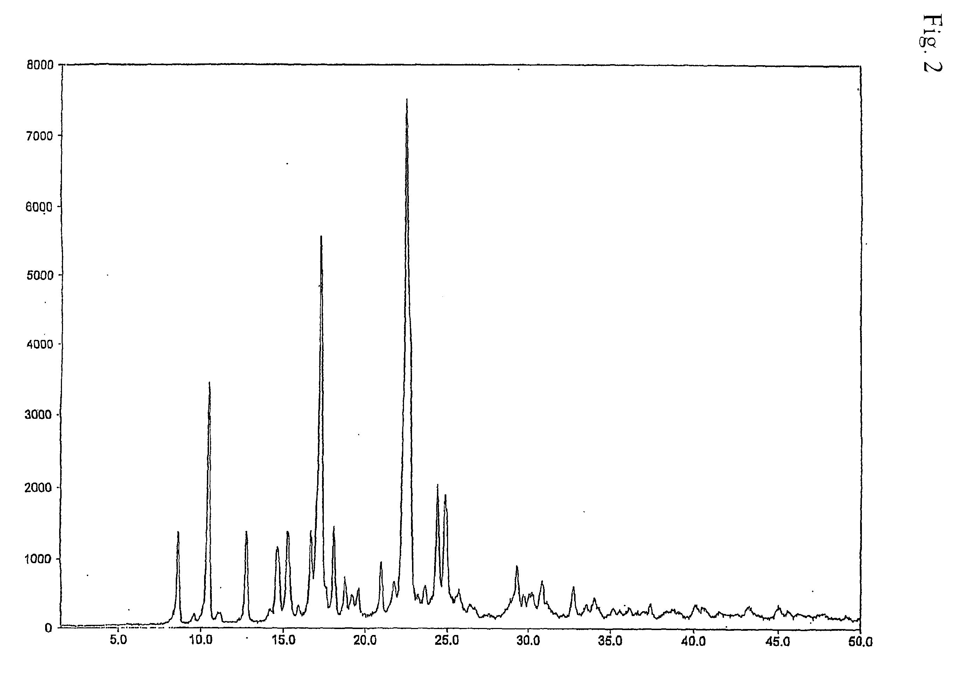

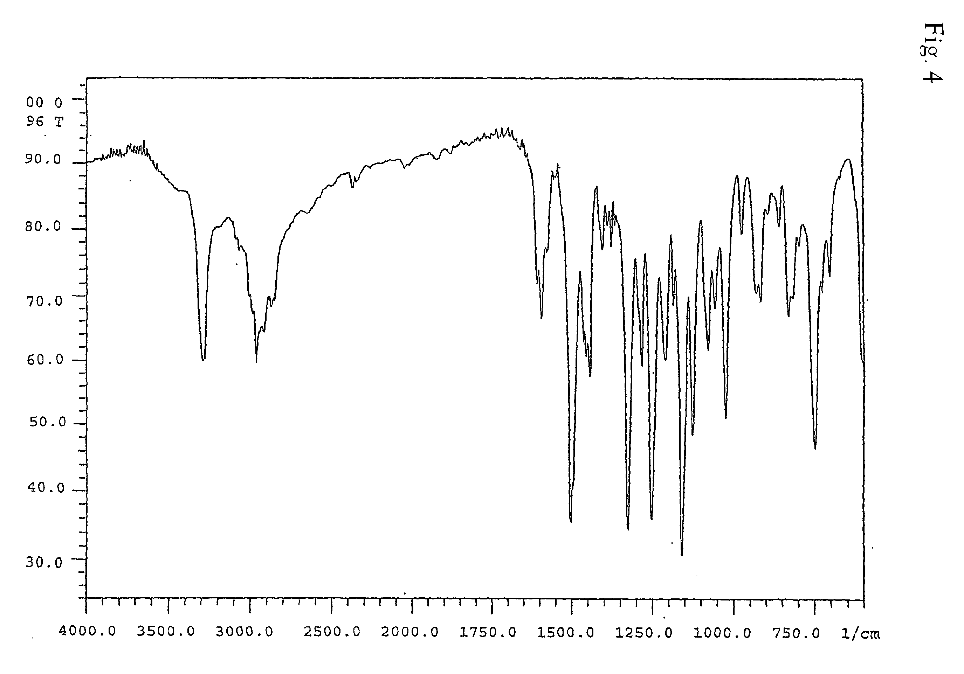

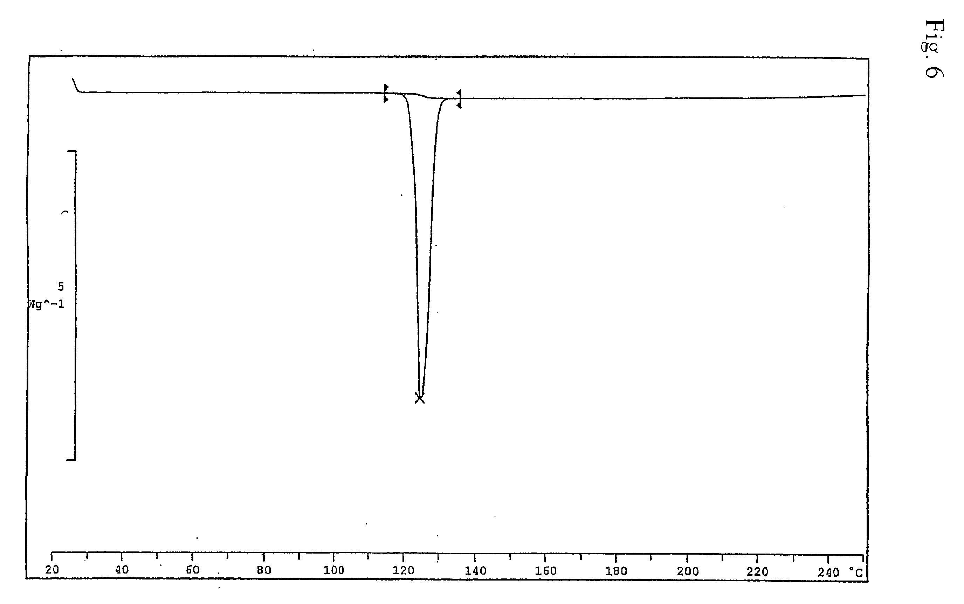

Another such modification is designated herein as Form 2. This Form of the free base has an X-ray diffraction pattern that substantially corresponds to Fig 2, an IR absorbance spectrum that substantially corresponds to Fig 4, and a DSC curve that substantially corresponds to Fig 6. A comparison of the X-ray diffraction patterns in Figs 1 and 2 shows that the tamsulosin free base Form 1 is different from the Tamsulosin free base Form 2. This difference is confirmed by the differences in IR spectra as well. Generally Form 2 has a melting point of about 124-126°C and Fig 6 shows a single melting endotherm for Form 2 of about 125°C. The Form 2 polymorph is obtainable, *inter alia*, by precipitation of tamsulosin base after neutralization of tamsulosin hydrochloride by a sodium hydroxide, in a methanol/water mixture, but is not limited thereto.

The solid state tamsulosin free base, especially the racemic free base, can be obtained surprisingly by precipitating the free base from a solution wherein the solvent for the solution comprises at least one of water or a lower alcohol. A "lower alcohol" means an alcohol having 1 to 4 carbon atoms and is preferably methanol or ethanol. Additional solvents may also be present along with the water and/or lower alcohol, including, without limitation, an ester such as ethyl acetate, an aliphatic ketone, such as acetone or methyl-isobutylketone, and/or an ether, especially a water-miscible ether such as dioxan or tetrahydrofuran. Preferred solvents are water, a mixture of water and methanol, and a mixture of ethyl acetate and methanol.

Precipitation can be carried out by any conventional technique including reducing the temperature of the solution, removing a portion of the solvent, etc. In some embodiments water can be used as a contra-solvent such that upon addition of the water, optionally in conjunction with a reduction in solution temperature, precipitation occurs. In a solvent system of water and methanol, tamsulosin free base precipitates in solid state from the solution at room temperature or less.

The tamsulosin free base precipitate can be recovered by filtration and optionally dried. Further, if desired, the solid tamsulosin free base may be recrystallized from a suitable solvent.

The solution of tamsulosin can be prepared or obtained in a variety of ways. For example, a residue from the synthesis of racemic tamsulosin can be dissolved in a solvent. A "residue" refers to the tamsulosin material obtained by evaporating off all or most of the solvent, for example, the solvent used in the synthesis of the tamsulosin molecule. For example, a racemic tamsulosin free base residue can be dissolved in water, typically at temperatures above room temperature, i.e. greater than 50°C, to form the solution from which racemic tamsulosin free base can be precipitated. Similarly, a racemic tamsulosin free base residue can be dissolved in a mixture of ethyl acetate and methanol to form the solution from which racemic tamsulosin free base can be precipitated. Alternatively, the tamsulosin free base solution can be formed as a result of its synthesis; i.e., the solvent in which the tamsulosin free base is obtained is appropriate as is or upon the addition of water and/or a lower alcohol for use in the inventive precipitation process.

Alternatively the solution of racemic tamsulosin can be formed from a solution of an acid addition salt of racemic tamsulosin. The process comprises treating an acid addition salt of racemic tamsulosin with a base in a solvent that comprises at least one of water or a lower alcohol to form the solution of racemic tamsulosin. The base can be an organic or inorganic base such as an alkali metal hydroxide especially sodium hydroxide, ammonia or an organic amine. The amount of base is preferably equimolar. The preferred range of reaction temperature is from room temperature to reflux temperature. The solvent in which the treating is carried out can be formed in *situ*, e.g., water can be supplied concurrently with the base to a solution of racemic tamsulosin free base in an organic solvent.

A particular embodiment of this process comprises treating an acid addition salt of tamsulosin in a solvent with base and precipitating tamsulosin free base from the solvent, wherein the solvent comprises water, a lower alcohol or both. This process is useful for both racemic tamsulosin such as racemic tamsulosin HCl as well as non-racemic and/or enantiomerically enriched camphor-10-sulfonate salts of tamsulosin. In either event, a solid free base form is obtained. The base and the treating conditions are the same as described above.

The tamsulosin salt-containing solution can be formed via the synthesis of tamsulosin or can be formed by dissolving an available solid form of the salt, e.g. a commercially available tamsulosin salt, in a solvent.

The precipitated tamsulosin free base is generally formed in high purity, typically at least 80%, more typically at least 90% and preferably at least 95% pure. This purity is based on the isolated and dried solid precipitate as is conventional in the art. If desired, the solid form tamsulosin free base can be recrystallized to improve its purity. In an example, the tamsulosin free base may be recrystallized from a solvent comprising a lower alcohol such as methanol, for instance from a mixture of methanol and ethyl acetate, or from a mixture of methanol and water.

Racemic tamsulosin or a salt thereof can be prepared by any suitable synthesis technique. Three preferred processes are described below in detail. In a first process ("Process A"), racemic tamsulosin free base may be prepared by reductive amination of the ketone of formula (4) by the amine of formula (5).

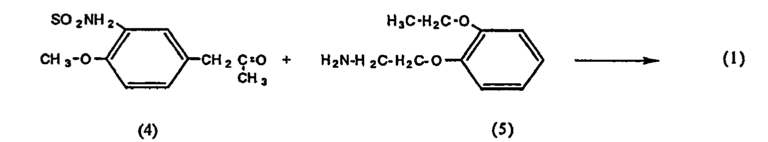

The (3-aminosulfonyl-4-methoxy)phenylacetone (4) is a novel compound and it may be prepared in analogy to a process described in US 544958 for related compounds. The starting compound is 4-methoxyphenyl acetone which is chlorosulfonated by chlorosulfonic acid at 0-5°C. After treating the reaction mixture with water, the resulting 3-chlorosulfonyl-4-methoxyphenylacetone (9) is formed in solid state and is isolated by filtration. The compound (9) is then treated in ethyl acetate solution with aqueous ammonia at 5°C. The formed crystals of crude (4) are recovered by filtration. The crude (4) may be purified by crystallisation, e.g. from ethanol.

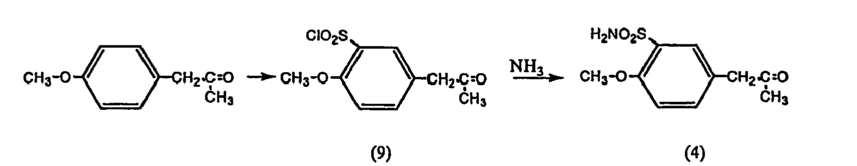

The 2-(o-ethoxyphenoxy)ethylamine (5) may be prepared according to a process described in BE 668124. In said process, 2-ethoxyphenol (10) reacts for 16-24 hours with chloroacetonitrile in refluxing acetone under presence of potassium carbonate. After filtration and evaporation of the solvent, the oily residue is dissolved in a suitable solvent such as ethanol or ether, treated with water and with a base such as ammonia or sodium hydroxide solution and crude o-ethoxyphenoxyacetonitrile (11) is obtained by evaporation of the solvent or by crystallization from the solvent. The crude (11) may be recrystallised from a suitable solvent, e.g. ethanol/water mixture. The compound (11) is catalytically hydrogenated under elevated pressure in a suitable solvent, e.g. toluene or toluene/triethylamine solution, using Raney cobalt or any other suitable catalyst. Crude free base of 2-(o-ethoxyphenoxy)ethylamine (5) is obtained after filtration and evaporation of the solvent as an oil.

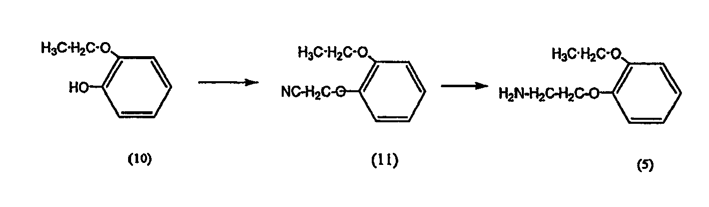

It has now been discovered that the so obtained free base of (5) is contaminated by side-products and should be purified for the next step. However, normal alkaline extraction surprisingly resulted in too high a loss. The amine (5) has unexpectedly high water solubility for an amine compound. It has been discovered that an effective way to purify the amine is to form a sulfonic acid salt of the amine, especially methane sulfonate or tosylate. The salt, particularly the methane sulfonate salt of (5), may be isolated in solid, preferably crystalline state by conventional methods and may be used as an advantageous substrate in the next reaction step. The conversion of the free base of (5) into an acid addition salt and isolation of the salt in solid state enhances the purity of the compound (5), particularly it removes side products that resulted from hydrogenation such as 2-ethoxyphenol. The 2-(o-ethoxyphenoxy)ethylamine methane sulfonate thus forms a specific aspect of this invention.

The racemic tamsulosin is obtained by reductive aminolysis of the ketone (4) with the free base of amine (5), for instance according to the process of US 4558156. If a salt of the amine (5) is used for the reaction, this salt is first converted to a free base by treatment with a suitable base, e.g. by sodium methoxide in methanol. An imino-compound is first prepared by contacting of both components in methanol. Hydrogenation catalyst such as platinum oxide or palladium/carbon is added to the reaction mixture and the mixture is hydrogenated by gaseous hydrogen, advantageously under enhanced pressure. After filtering off the catalyst, the reaction mixture is preferably acidified with an acid, preferably with alcoholic or aqueous HCl, thereby forming an acid addition salt of racemic tamsulosin, preferably tamsulosin hydrochloride. The racemic tamsulosin salt is isolated in solid form, preferably crystalline form by evaporation of the solvent or by crystallization from the solvent. The racemic tamsulosin salt is then treated with base and tamsulosin free base is precipitated as described above.

In a second process ("Process B"), racemic tamsulosin hydrochloride may be prepared in a form of a hydrochloride salt from hydroxy-tamsulosin of formula (8) according to a method of EP 34432. Starting hydroxy-tamsulosin (8) may be prepared from the amine compound (5) according to US 4,217,305. The process comprises, in a first step, conversion of the hydrochloride salt of hydroxy-tamsulosin (8) into a chloro-tamsulosin hydrochloride (12) by means of reaction of (8) with thionylchloride in acetonitrile, and isolation of the product from the reaction mixture after its spontaneous crystallization from the reaction mixture. The compound (12) is subsequently dehalogenated by, e.g., catalytic hydrogenation using palladium on carbon as the catalyst at normal temperature and pressure. After concentration of the reaction mixture, crystalline tamsulosin hydrochloride is obtained and it may be recrystallized from a mixture of methanol and ethanol.

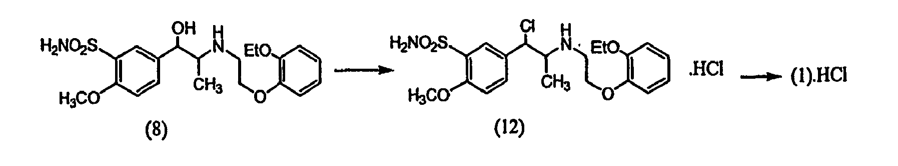

To obtain racemic tamsulosin free base in solid state, the procedure of the present invention disclosed above should be applied.

In a third process ("Process C"), racemic tamsulosin free base may be prepared by condensation of the racemic amine of the formula (6) with a bromo-compound of 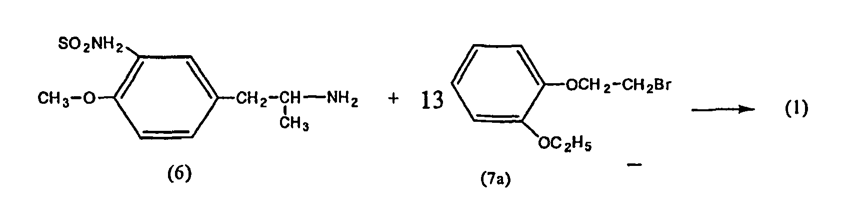 the formula (7a) in refluxing methanol. The conditions of the manufacturing procedure were disclosed in US 5447958, however, the starting amine (6) used therein was optically active. Tamsulosin free base (optically active) was obtained after separation of the reaction mixture by column chromatography in solid state and converted to hydrochloride. The column chromatography however makes this process impractical for industrial scale production of racemic tamsulosin free base.

However, it has been discovered that the process should be modified in such a way that a dipolar aprotic solvent such as dimethylformamide is used for the condensation of racemic amine (6) with (7a) and the reaction temperature is between 70 and 100°C. After removal of the solvent by distillation under reduced pressure, hot water is added to the solid residue and tamsulosin free base is allowed to crystallize by decreasing the temperature. The obtained solid product may be further purified by extraction of the side products by hot water. Optionally, tamsulosin free base may be converted to tamsulosin hydrochloride by methods known per se and recovered back from the hydrochloride by a process given above. Starting reactants may be prepared by methods known in the art.

Alternatively, the tamsulosin free base may be prepared from the racemic amine (6) by reductive amination of the aldehyde (7b); the manufacturing conditions including the synthesis of the starting aldehyde are described in AT 397960.

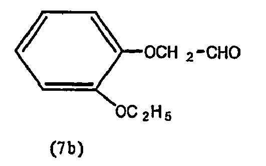

The solid state racemic tamsulosin base is useful in a novel process of optical resolution of tamsulosin into enantiomers, especially into pure (R)-tamsulosin, based on fractional crystallization of a pair of diastereomeric salts. Making racemic tamsulosin and resolving the racemate into a pure or substantially pure enantiomer can offer benefits over the use of optically pure synthesis schemes to make (R)-tamsulosin.

In general, the novel resolution process involves converting the solid state racemic tamsulosin base into a diastereomeric salt pair by contacting the substrate in a suitable solvent with a suitable optically active (i.e., chiral) sulfonic acid. The chiral sulfonic acids useful with the present invention are preferably monovalent organic sulfonic acids having pKa value lower than about 3.5. Preferred chiral acids are camphor sulfonic acids including lower alkyl and/or halo derivatives thereof. Specifically preferred acids are (-)-camphor-10-sulfonic acid and (+)-camphor-10-sulfonic acid. These acids are commercially available and can be made by methods well known in the art.

The amount of the chiral acid used in forming the diastereomeric pair is typically within the range of 0.5 - 2 moles per 1 mole of tamsulosin and is preferably essentially equimolar.

The solvent is selected so as to facilitate the salt reaction and preferably to allow subsequent separation of the resulting diastereomers by fractional crystallization. In the process, a mixture of tamsulosin substrate free base with a solvent may be contacted with a solid chiral acid, or a mixture of chiral acid with a solvent may be contacted with solid tamsulosin base, or both partners may be combined with a solvent prior to being contacted together. The contact may be made using a single solvent or a mixture of solvents. Normally the substrate and the acid are dissolved in the solvent, even if they started as a solid at the beginning of the contact, in order to facilitate an efficient salt reaction. Suitable solvents include lower alcohols especially methanol and ethanol, acetone, dioxane, ethyl acetate, mixtures thereof, and mixtures of one or more of these solvents with water. Preferred solvents are methanol and methanol-water mixtures.

The temperature of contact is from ambient to the boiling point of the solvent system, the later being preferred. It is not required that a complete solution is formed in this step, though it is preferred.

The salt reaction forms a pair of diastereomers: one diastereomer resulting from the reaction of (R)-tamsulosin with the chiral sulfonic acid and another resulting from the reaction of the (S)-tamsulosin with the chiral sulfonic acid. One of the diastereomers is preferentially precipitated from the solvent. The precipitation is "preferential" in that the conditions used allow for one of the diastereomers to be precipitated to a greater extent than the other. The precipitation of the solid phase may be spontaneous, or may be induced by changing the conditions of the solution, e.g. by cooling the mixture after contact, adding a contra-solvent, removal of a part of the solvent or by combination of these techniques. As used herein "induced" includes partially induced such as where some precipitation occurs spontaneously and more precipitation is achieved by an inducement technique as well as precipitation achieved by only an inducement technique. The precipitation, whether spontaneous or induced, may also be facilitated by the presence of or inoculation with a seeding crystal of the desired salt.

The obtained solid salt is substantially enriched by one enantiomer of tamsulosin, advantageously by the (R)-tamsulosin. As used herein "enrichment" means that the product contains more of one of the (R)- or (S)- tamsulosin or tamsulosin diastereomer than the starting substrate or composition. For example, if the starting tamsulosin contained a 50:50 mixture of (R) and (S) enantiomers, then a precipitation of a salt having an (S):(R) tamsulosin ratio of 30:70 would be a diastereomeric enriched precipitate because the diastereomer having (or derived from) the (R)-tamsulosin has been increased relative to the amount in the initial solution. Similarly, the mother liquor is likewise enriched by the other diastereomeric salt formed from the second enantiomer, e.g. (S)-tamsulosin, and thus is a diastereomeric enriched solute.

In a preferred variant, the diastereomeric salt of the desired (R) isomer of tamsulosin with a chiral acid is less soluble than that of diastereomer of the (S) isomer and thus the (R) form preferentially precipitates out of the solution. The precipitate may be separated from the reaction mixture by ordinary methods such as by filtration or centrifugation.

In another variant, the salt of the desired (R)-tamsulosin isomer with a chiral acid is more soluble than that of the (S) isomer and it remains in the solution after separation of the solid. Then, the mother liquor contains the desired enantiomer of tamsulosin and may be elaborated by various ways. For instance, the solvent may be evaporated or a contrasolvent may be added to obtain the desired salt in solid state. Advantageously, the salt is not isolated in a solid state and the obtained solution is used as such in the next step of liberation of (R)-tamsulosin from the salt. Optical yields of this variant are similar to those as above.

In an example of the advantageous embodiment of the optical resolution process of our invention, substantially racemic tamsulosin reacts with (-)-camphor-10-sulfonic acid in a methanol whereby (R)-tamsulosin (-)camphor-10-sulfonate separates out from the solution as a solid, while the salt of the (S) enantiomer remains in the solution. The (R) salt may be separated from the reaction mixture after precipitation by ordinary methods e.g. by filtration or centrifugation.

The salt substantially enriched by the desired enantiomer of tamsulosin, either the precipitate or the solute, is elaborated in the next step to liberate the so enriched tamsulosin from the salt form. The liberation step essentially comprises treatment of the salt (in solid, suspended or dissolved state) with an organic or inorganic base; such base must be stronger than the basicity of tamsulosin.

The organic or inorganic base liberates both the tamsulosin and the used optically active acid from their mutual salt form and forms a new salt with such acid, while tamsulosin substantially enriched by the desired enantiomer is obtained as a free base.

The liberation step is advantageously performed in a solvent which at least partially dissolves the used salt and base. Generally, the liberation of the desired enantiomer of tamsulosin from the enriched salt proceeds by contacting the salt with an equivalent of a suitable base, e.g., metal hydroxides, in a proper solvent, advantageously in water. The so formed free base of the enriched tamsulosin is isolated by ordinary methods. If water has been employed as a solvent for neutralization, the tamsulosin base precipitates as a solid and is isolated by filtration or centrifugation.

In a preferred aspect, the obtained product comprises substantially the (R) isomer of tamsulosin. The formed free base of tamsulosin, especially the enriched (R) enantiomer, may be further converted into an acid addition salt with a suitable acid, particularly with a pharmaceutically acceptable acid, by methods known per se. Examples of such salts are the hydrochloride, hydrobromide, acetate, fumarate, maleate, citrate or methane sulfonate.

If the optical purity of the obtained tamsulosin product is not sufficient, the resolution may be enhanced by repeating the process. Accordingly, a second pair of diastereomeric tamsulosin salts can be the same or different as the first pair. In one embodiment, the second pair is different than the first such as by using an optically active sulfonic acid that is different in rotation from the first acid. In certain embodiments, it is advantageous that the first precipitation preferentially precipitates the diastereomer containing the (S)-tamsulosin and the second precipitation from a second solvent preferentially precipitates the (R)-tamsulosin containing diastereomer. In this embodiment, the enriched solute from the first precipitation is used either with or without liberation of the tamsulosin free base to make a solution for the second precipitation.

Optically pure or substantially pure (R)-tamsulosin, acid addition salts thereof and particularly (R)-tamsulosin hydrochloride are useful in the preparation of medicaments for treatment of various diseases or conditions including cardiac insufficiencies and benign prostatic hyperplasia among others. It may be used alone or in combination with other active components. Such medicaments may be formulated for peroral, transdermal or parenteral application, for instance in a form of tablets or capsules. The formulations comprise therapeutically effective amounts of the active substance together with a pharmaceutically acceptable carriers or diluents and may be prepared by any conventional method.

The present invention is more particularly described and explained by the following examples. It is to be understood, however, that the present invention is not limited to these examples and various changes and modifications may be made without departing from the scope of the present invention.

### Example 1

### Synthesis of racemic tamsulosin free base

### a) Synthesis of 2-Methoxy-5-(2-oxopropyl)benzenesulfonamide (4)

Chlorosulfonic acid (426 g, 3.656 mol) is cooled down to -10 - (-15)°C. 4-methoxyphenylacetone (100 g, 0.609 mol) is added in such a rate as not to exceed temperature 5°C in reaction mixture. After addition of all amount of methoxyphenylacetone the reaction mixture is allowed to warm up to room temperature. Mixture is stirred for 2 hours at room temperature. The reaction mixture is then poured on stirred mixture of ice (1500 g) and water (1600 ml). Formed crystals are filtered, washed with cold water (200 ml).

The crystals are dissolved in ethyl acetate (300 ml). Aqueous ammonia (600 ml) is cooled down to -5°C and the above ethyl acetate solution is gradually added in such a rate as not to exceed 5°C. The mixture is then allowed to warm to room temperature and stirred overnight. Formed crystals are filtered, washed with water (200 ml) and ethanol (100 ml). Crystals of the crude product were recrystallized from ethanol to give 65 g of title compound.

### b) Synthesis of (2-Ethoxyphenoxy)methyl cyanide (11)

Potassium carbonate (550 g, 3.98 mol) was added to acetone (1800 ml) and resulting suspension was stirred for 30 min. 2-Ethoxyphenol (460 g, 3.329 mol) was gradually added under stirring. The mixture was heated to reflux. Chloroacetonitrile (275 g, 3.642 mol) was added and the mixture was stirred under reflux for 24 hours. The reaction mixture was cooled down to room temperature. Solid was filtered off, washed with acetone (750 ml) and combined filtrates were evaporated to give oil. The oil was dissolved in ethanol (180 ml), the solution was heated to reflux and mixture of water (530 ml) and aqueous ammonia (45 ml) was added. The mixture was cooled to 5 - 10°C under stirring. Crude crystalline product was filtered, washed with mixture of ethanol (250 ml) and water (400 ml). The crude product was recrystallized from ethanol/water mixture to give pure product (500 g).

### c) Synthesis of 2-(2-Ethoxyphenoxy)-1-ethanamine (5) methanesulfonate

(2-Ethoxyphenoxy)methyl cyanide (400 g, 2.257 mol) was dissolved in toluene (750 ml) and the solution was transferred to autoclave. 125 g of Raney cobalt was added to autoclave and the mixture was hydrogenated at 30-40°C and hydrogen pressure 1.7 to 1.2 Mpa for one hour. The catalyst was removed by filtration and the filtrate was evaporated to give an oil.

The oil was dissolved in ethyl acetate (550 ml) and methanesulfonic acid (150 g) was added under stirring. The temperature was maintained between 20-25°C. Formed crystals were filtered, washed with ethyl acetate (250 ml) and dried 40°C to give 430 g of the title product.

### d) Synthesis of racemic tamsulosin hydrochloride :

2-(2-Ethoxyphenoxy)-1-ethanamine methanesulfonate (300 g, 1.08 mol) is dissolved in methanol (1 000 ml) at 40-50°C. Solution of sodium methoxide (30% soln, 195 g) is added under stirring. The mixture is cooled to 20-15°C. Formed sodium methanesulfonate is filtered off, and washed on filter with methanol (2 x 100 ml). Combined filtrates are transferred to autoclave. 2-Methoxy-5-(2-oxopropyl)benzenesulfonamide (263.3 g, 1.08 mol) is added and the suspension is stirred for 20 min. Catalyst Pt/C (5%Pt, 60 g) is added and the mixture is hydrogenated at 50-56°C and at hydrogen pressure 1.7 to 1.2 Mpa for 1.5 hour. The catalyst was removed by filtration and hydrochloric acid (37%, 90 g) was added to the filtrate under stirring. Formed crystals of tamsulosin hydrochloride were filtered, washed with methanol (500 ml) and dried at 50°C to give 360 g of title compound.

### e) Synthesis of racemic tamsulosin free base

The crystals obtained in step d) were suspended in methanol (1 100 ml), the mixture was heated to reflux and sodium hydroxide solution (2M, 440 ml) was gradually added followed by water (350 ml). The mixture was cooled down to 10-15°C. Formed crystals were filtered and washed on filter with methanol (150 ml) and water (150 ml) mixture. Crystals were dried at 50°C to give 340 g of product.

### Example 2A

### Synthesis of racemic tamsulosin free base

5-(-2-aminopropyl]-2-methoxybenzenesulfonamide (200 g) was dissolved in dimethylformamide (950 ml) and 1-(2-Bromoethoxy)-2-ethoxybenzene (100.3 g) was added. The reaction mixture was heated to 80-85°C for 4 hours. Dimethylformamide was then distilled off under vacuum. Water (1000 ml) was added to solid residue and the mixture was heated to 80-90°C under stirring for 2 hours. The mixture was cooled to room temperature. Formed crystals were filtered off and were suspended in water (900 ml). Suspension was heated to 80-90°C under stirring for 2 hours. Crystals were filtered, washed with water (200 ml) and dried to give tamsulosin base (150 g, 89.8%).

### Example 2B

### Purification of racemic tamsulosin free base

53.3. g of tamsulosin free base was dissolved, under reflux in 370 ml of a ethyl acetate/ methanol mixture (56 : 44 w/w). The obtained solution was allowed to cool, under formation of a precipitate. The mixture was cooled to 15-20°C and stirred 30 minutes at this temperature. The solid was isolated by filtration, washed with the solvent mixture and dried. Yield : 45.3. g. Solid state properties of the product correspond to Form 1.

### Example 2C

### Purification of racemic tamsulosin free base

Tamsulosin free base (159 g) (purity 94%) was suspended in a methanol (280 ml)/ water mixture (280 ml). The mixture was heated to reflux until all material dissolved. Hydrochloric acid (37%, 44 g) was added and the mixture was gradually cooled down to 0°C. Formed crystals were filtered off, washed with cold methanol (70 ml). Wet crystals were suspended in methanol (450 ml) and the mixture was heated to reflux. Aqueous sodium hydroxide (2M, 150 ml) was then added. The mixture was cooled down and water (140 ml) was added. Formed crystals were filtered off, washed with methanol-water mixture (1:1, 100 ml) and dried. Yield 117 g of a crystalline product (70%, purity 99.7 %). Solid state properties of the product correspond to Form 2.

### Identification of tamsulosine free base

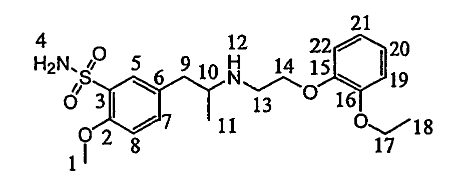

### ¹H-NMR spectrum:

The ¹H-NMR spectrum was measured at 303.2 K on a Bruker Avance-400 in deuterated dimethylsulfoxide at 400 MHz.

| δ | assignment |
|---|---|
| 0.95 | (d, 3H, *J_{10,11}*=6.3Hz, H-11); |
| 1.30 | (t, 3H, *J_{17,18}*=7.0Hz, H-18); |
| 2.48 | (dd, 1H, *J_{9,10}*=7.3Hz, *J_{9,9}*=13.4Hz, 1 x H-9); |
| 2.77 | (dd, 1H, *J_{9,10}*=5.3Hz, *J_{9,9}*=13.4Hz, 1 x H-9); |
| 2.90 | (m, ~3H, H-10 + H-13); |
| 3.89 | (s, 3H, H-1); |
| 4.01 | (m, 4H, H-14 + H-17); |
| 6.89 | (m, 2H, H-20 + H-21); |
| 6.96 | (m, ~2H, H-19 + H-22); |
| 7.01 | (bs, 1-2H, H-4); |
| 7.11 | (d, 1H, *J_{7,8}*=8.6Hz, H-8); |
| 7.40 | (dd, 1H, *J_{5,7}*=2.3Hz, *J_{7,8}*=8.6Hz, H-7); |
| 7.58 | (d, 1H, *J_{5,6}*=2.3Hz, H-5). |

### ¹³C-NMR spectrum:

The ¹³C - NMR spectrum was measured at 303.2 K on a Bruker Avance-400 in deuterated dimethylsulfoxide at 100.6 MHz.

| δ | assignment |
|---|---|
| 14.66 | (C-18); |
| 19.66 | (C-11); |
| 41.25 | (C-9); |
| 45.56 | (C-13); |
| 53.87 | (C-10); |
| 55.92 | (C-1); |
| 63.87 | (C-17); |
| 69.01 | (C-14); |
| 112.32 | (C-8); |
| 114.09, 114.68 | (C-19, C-22); |
| 120.88, 121.19 | (C-20, C-21); |
| 127.96 | (C-5); |
| 130.82,131.09 | (C-3, C-6); |
| 134.11 | (C-7); |
| 148.38,148.56 | (C-15, C-16); |
| 154.18 | (C-2). |

### Example 3

### Resolution of tamsulosin free base with (-) camphor-10-sulfonic acid

100 mg of racemic tamsulosin free base and 58 mg of (-)camphor-10-sulphonic acid were dissolved in 12 ml of ethanol while heating. The solution was allowed to cool to room temperature and stored overnight. The formed solid was filtered off, washed with 1 ml of ethanol and 2 ml of ether and dried.

Optical purity (HPLC) : 61.3% of R-tamsulosin (-) camphor-10-sulfonate. After recrystallization from ethanol, the optical purity increased to 68.7%.

### Example 4

### Resolution of tamsulosin free base with (+)camphor-10-sulphonic acid

2.0 g of racemic tamsulosin free base and 1.71 g of (+)camphor-10-sulphonic acid was dissolved in 45 ml of methanol at reflux. The solution was slowly cooled up to 4C. The formed crystals were collected by filtration. Optical purity (HPLC) : 75% of (S)-tamsulosin-(+)camphor-10-sulphonate.

640 mg of the product were recrystallised from 5 ml of methanol. After standing overnight, the formed crystals were collected by filtration and dried. Optical purity (HPLC) : 94% of (S)-tamsulosin-(+)camphor-10-sulphonate.

300 mg of the product were recrystallised form 3 ml of methanol. After standing overnight at 30C, the formed crystals were collected by filtration, washed with ethanol and dried. Optical purity (HPLC): 96.5% of (S)-tamsulosin-(+)camphor-10-sulphonate.

### Example 5

### Resolution of racemic tamsulosin base by a process employing combination of (+)- and (-) camphor-10-sulfonic acids.

**a)** 1200 g of racemic tamsulosin free base was suspended in 4700 ml of methanol and the mixture was heated to reflux. A solution of 682.4 g of (+)-camphor-10-sulfonic acid in 4700 ml of water was added to the mixture. Resulted mixture was heated to reflux and allowed to cool under stirring. At about 45°C, a solid started to precipitate. The mixture was cooled to 20-25°C a stirred at this temperature for 5 hours. Crystalline solid was filtered out, washed with 200 ml of cold (0°C) methanol and dried.
The solid product was suspended in 3050 ml of 50% aqueous methanol, heated to reflux and allowed to cool under stirring. After a solid began to precipitate (at approx. 55°C), the mixture was cooled to 20-25°C and stirred for 5 hours. The crystalline product was filtered out, washed with 150 ml of cold (0°C) methanol and dried. Yield: 693.4 g of (S)-tamsulosin (+) camphor-10-sulfonate.
**b)** Mother liquors from both crystallizations were collected and 1020 ml of 2N aqueous solution of NaOH was added under stirring until the mixture was the slightly alkaline (pH 9-10). The resulting suspension of tamsulosin base was cooled to 0-5°C for 2 hours, filtered, washed with water and dried.
Yield: 697.5 g of tamsulosin free base comprising 76% of the (R)-enantiomer.
**c)** The obtained tamsulosin free base was suspended in 1920ml of methanol and heated to reflux. A solution of 396.6 g of (-)camphor-10sulfonic acid in 1920 ml of water was added to the boiling suspension. Reaction mixture was heated to reflux and allowed to cool under stirring. After a solid started to precipitate (approx. 35°C), the suspension was cooled to 20-25°C and stirred for 5 hours. Crystalline precipitate was filtered out, washed with cold methanol and dried.
Yield : 806.8 g of tamsulosin (-)camphor-10-sulfonate comprising 89.7% of the (R) enantiomer.
**d)** Recrystallization of raw (R)-tamsulosin (-)camphor-10-sulphonate General procedure:
Raw (R)-tamsulosin (-)camphor-10-sulphonate was dissolved under stirring in 3.5parts (by volume) of 50% aqueous methanol at reflux. The solution was allowed to cool until a solid began to separate and then cooled to 20-25°C. The mixture was stirred for 5 hours and the solid was separated by filtration. The solid was washed by 1.5 parts (by volume) of cold (0°C) methanol and dried. The crystallization process was repeated several times with the following results :

| Crystallization No | Yield of crystallization | Content of R-isomer |
|---|---|---|
| 0 | | 89.7% |
| 1 | 63.6% | 95.7% |
| 2 | 56.9% | 98.4% |
| 3 | 51.7% | 99.5% |
| 4 | 47.4% | 99.8% |

The product of the last crystallization had a melting range of 208-211 C and optical rotation of -17.2° ( c=0.5 in methanol). IR spectrum in KBr exhibits peaks of, i.a., 1740, 1505,1161 or 1044 cm⁻¹. Its identity was proven by NMR spectrum.

### Example 6

### Conversion of (R)-tamsulosin(-)camphor-10-sulfonate to (R)-tamsulosin free base

518.3 g of (R)-tamsulosin (-) camphor-10-sulfonate (opt. purity 99.8%) was dissolved under reflux in 3100ml of 50% aqueous methanol and 445 ml of 2N aqueous NaOH was added while hot. The resulted suspension was cooled to 0-5°C for 2 hours. The solid was filtered out, washed by water and dried. Yield: 315 g of (R)-(-)-tamsulosin free base containing 99.9% of R-isomer.

The product was dissolved under reflux in 3500 ml of 50% aqueous methanol and allowed to cool to 20-25°C under stirring. The suspension was stirred for 8 hours. The solid was filtered off, washed with 500ml of water and dried. Yield: 309.11 g (R)-(-)-tamsulosin free base comprising more than 99.9% of R-isomer.

### Example 7

### Conversion of (R)-tamsulosin free base into (R)-tamsulosin hydrochloride

309.11 g of (R)-(-)-tamsulosin free base was suspended in 1080 ml of 50%aqueous methanol, heated to reflux and treated with 125 ml of concentrated hydrochloric acid under stirring. The resulted solution was cooled, whereby a solid crystallized. Resulted suspension was cooled to 0-5°C for 1.5 hours. Solid product was filtered off, washed with 500 ml of cold (0°C) methanol and dried. Yield: 320 g of (R)-(-)-tamsulosin hydrochloride comprising more than 99.9% of R-isomer.

The invention having been thus described, it will be obvious to the worker skilled in the art that the same may be varied in many ways without departing from the spirit of the invention and all such modifications are included within the scope of the present invention as set forth in the following claims.

## Claims

1. A process for isolation of tamsulosin free base in solid state which comprises treating an acid addition salt of tamsulosin in a solvent with a base and precipitating tamsulosin free base from said solvent, wherein said solvent comprises water.

2. The process according to claim 1 wherein the solvent further comprises a C1-C4 alcohol.

3. The process according to claim 2 wherein the solvent is a water and methanol mixture.

4. The process according to any of the claims 1-3, wherein said acid addition salt of tamsulosin is tamsulosin camphor-10-sulfonate salt.

5. The process according to any of the claims 1-3 wherein said acid addition salt of tamsulosin is tamsulosin hydrochloride.

6. Tamsulosin free base obtainable according to the process of any of the claims 1-5.

7. A sulfonic acid addition salt of 2-(o-ethoxyphenoxy)ethylamine.

8. The sulfonic acid addition salt according to claim 7, which is 2-(o-ethoxyphenoxy)ethylamine methane sulfonate or 2-(o-ethoxyphenoxy)ethylamine tosylate.
